# EUROPÄISCHE PATENTSCHRIFT

(11) **EP 2 185 217 B1**
(45) Veröffentlichungstag und Bekanntmachung des Hinweises auf die Patenterteilung: **23.11.2016**
(21) Anmeldenummer: 08785665.4
(22) Anmeldetag: 22.08.2008
(51) Int. Cl.: A61M 5/00, A61M 5/34

(54) **RÖNTGENEINRICHTUNG UND RÖNTGENVERFAHREN**
X-RAY DEVICE AND METHOD
DISPOSITIF ET PROCÉDÉ À RAYONS X

(30) Priorität: 28.08.2007 DE 102007040488
(43) Veröffentlichungstag der Anmeldung: 19.05.2010
(73) Patentinhaber: Arzneimittel GmbH Apotheker Vetter & Co. Ravensburg, 88212 Ravensburg (DE)
(72) Erfinder: GLOCKER, Joachim, 88250 Weingarten (DE); PETERS, Dirk, 88255 Baienfurt (DE)
(74) Vertreter: Gleiss, Alf-Olav
(86) Internationale Anmeldenummer: PCT/EP2008/006902
(87) Internationale Veröffentlichungsnummer: WO 2009/030379

(56) Entgegenhaltungen:
- DE-C1- 19 806 971
- US-A- 5 500 886
- US-A- 5 590 170
- US-A- 5 909 478
- US-A1- 2004 168 293
- US-A1- 2007 147 583

## Beschreibung

Die Erfindung betrifft eine Röntgeneinrichtung zur Untersuchung von eine Kanüle aufweisenden Spritzenkappen gemäß Oberbegriff des Anspruchs 1 sowie ein Verfahren zur Untersuchung von eine Kanüle aufweisenden Spritzenkappen gemäß Oberbegriff des Anspruchs 17.

Röntgeneinrichtungen und Verfahren der hier angesprochenen Art sind bekannt. Sie dienen der Untersuchung von Spritzenkappen, die eine Kanüle enthalten. Derartige Kappen werden auf Spritzen mit einer Kanüle aufgesetzt, einerseits um die Spritze mit der Kanüle steril abzudecken, andererseits um einen Schutz vor Verletzungen zu bieten. Spritzenkappen dieser Art schließen häufig einen elastischen Stopfen ein, in den die Kanüle einsticht. Eine Spritzenkappe der hier angesprochenen Art kann auf eine Spritze aufgesetzt werden, die bereits mit einer Kanüle versehen ist. Diese kann beim Montieren am Spritzenkörper verbiegen oder dort schief eingesetzt werden, sodass sie dann beim Einstechen in den Stopfen bereits schief ist. Eine Spritzenkappe der hier angesprochenen Art kann aber auch mit einer Kanüle versehen, also quasi vormontiert sein, und dann auf eine Spritze aufgesetzt werden. Auch bei der Vormontage kann die Kanüle schief in den Stopfen eingeführt oder beim Einstecken verbogen werden. Röntgeneinrichtungen der hier angesprochenen Art dienen dazu, in der Spritzenkappe schief sitzende Kanülen zu erfassen. Sie weisen zwei Röntgenquellen auf, welche die Spritzenkappe aus zwei Richtungen, vorzugsweise aus zwei um 45° bis 90° versetzten Richtungen, durchleuchten. Anhand der beiden erzeugten Bilder kann dann festgestellt werden, ob die Kanüle innerhalb der Spritzenkappe schief angeordnet ist. Ab einer bestimmten Winkelstellung werden Spritzenkappen ausgesondert, um zu vermeiden, dass die Spitze der Kanüle möglicherweise die Wand der Spritzenkappe durchsticht, was einerseits dazu führt, dass die Sterilität der Kanüle nicht mehr gegeben ist, andererseits dazu, dass eine Verletzungsgefahr gegeben ist. Röntgeneinrichtungen der hier angesprochenen Art sind teuer, weil zum Einen zwei Strahlungsquellen und zum Anderen eine Bildauswertungsschaltung erforderlich sind, die die Bilder der beiden Strahlungsquellen auswertet.

Aufgabe der Erfindung ist es daher, eine Röntgeneinrichtung zu schaffen, die einfach aufgebaut und kostengünstig realisierbar ist.

Zur Lösung dieser Aufgabe wird eine Röntgeneinrichtung zur Untersuchung von eine Kanüle aufweisenden Spritzenkappen vorgeschlagen, welche die in Anspruch 1 genannten Merkmale aufweist, nämlich eine Röntgenstrahlquelle, einen Röntgendetektor und eine Halterung. Die Röntgeneinrichtung zeichnet sich dadurch aus, dass die Spritzen kappe so im Strahlengang angeordnet ist, dass ihre Längsachse mit der Hauptachse des Strahlengangs zusammenfällt. Die Spritzenkappe wird also von oben oder von unten mit Röntgenstrahlen beaufschlagt, um die Position der Kanüle zu kontrollieren. Wird bei einer derartigen Anordnung der Spritzen kappe im Strahlengang durch den Röntgendetektor an der Position, an der die Kanüle erwartet wird, ein Punkt festgestellt, so ist die Kanüle konzentrisch oder koaxial zum Strahlengang ausgerichtet. Sie befindet sich also auch koaxial zur Längsachse der Spritzenkappe. Für diese Art der Untersuchung ist es gleichgültig, ob die Kanüle in einer separaten Spritzenkappe untergebracht ist und gemeinsam mit dieser auf einer Spritze aufgesetzt wird, oder ob die Spritzenkappe auf einer Spritze aufgesetzt ist, welche die Kanüle umfasst. Wird aber die Kanüle als Strich im Röntgendetektor abgebildet, so ist davon auszugehen, dass die Kanüle nicht exakt in der Längsachse der Spritzenkappe liegt, sondern unter einem Winkel dazu. In diesem Fall wird die Kanüle ausgesondert.

Bei einem bevorzugten Ausführungsbeispiel der Röntgeneinrichtung ist vorgesehen, dass zwischen Röntgenstrahlungsquelle und Untersuchungsort mindestens ein Kollimator im Strahlengang angeordnet ist. Dieser dient dazu, die Belastung des um den Untersuchungsort angeordneten Bereichs durch Strahlen auf ein Minimum zu reduzieren.

Besonders bevorzugt wird ein Ausführungsbeispiel der Röntgeneinrichtung, das sich dadurch auszeichnet, dass zusätzlich ein erstes Referenzelement vorgesehen ist. Dieses dient dazu, bei der Erzeugung eines Abbildes der Spritzenkappe im Röntgendetektor ein erstes Referenzsignal zu haben. Vorzugsweise weist das Referenzelement eine kreisförmige Öffnung auf.

Besonders bevorzugt wird ein Ausführungsbeispiel der Röntgeneinrichtung, die sich durch ein zweites Referenzelement auszeichnet, das vorzugsweise ringförmig ausgebildet ist. Auch dieses dient dazu, ein Referenzsignal im Röntgendetektor zu erzeugen. Durch die beiden Referenzsignale ist es besonders einfach möglich zu entscheiden, ob die Kanüle schief verläuft, also unter einem Winkel zur Längsachse der Spritzenkappe, und ob die Schiefstellung noch akzeptabel ist.

Weitere Ausgestaltungen der Röntgeneinrichtung ergeben sich aus den Unteransprüchen.

Aufgabe der Erfindung ist es außerdem, ein Verfahren zur Untersuchung von eine Kanüle aufweisenden Spritzenkappen zu schaffen, welches einfach und kostengünstig realisierbar ist.

Zur Lösung dieser Aufgabe wird ein Verfahren der oben genannten Art vorgeschlagen, welches die in Anspruch 17 genannten Merkmale aufweist. Bei diesem Verfahren wird eine Spritzen kappe im Strahlengang zwischen einer Röntgenstrahlungsquelle und einem Röntgendetektor so angeordnet, dass die Längsachse der Spritzenkappe der Hauptachse des Strahlengangs zwischen Röntgenstrahlungsquelle und Röntgendetektor zusammenfällt. Die Spritzenkappe wird also in Richtung ihrer Längsachse durchleuchtet. Mittels des Röntgendetektors wird dabei zumindest ein eine Abbildung der Kanüle zeigendes Röntgenbild erzeugt. Ein Vergleich der Form oder Größe der Kanülenabbildung mit einer noch ein akzeptables Maß einer Auslenkung der Kanüle gegenüber der Längsachse der Spritzenkappe definierenden Referenzabbildung zeigt, ob eine Schrägstellung der Kanüle gegenüber der Längsachse der Spritzenkappe noch in einem hinnehmbaren Bereich liegt. Besonders vorteilhaft an der Durchleuchtung der Spritzenkappe in Längsrichtung ist, dass jede Schrägstellung der Kanüle erkannt wird, also unabhängig davon, in welcher Richtung die Kanüle in Bezug auf eine Röntgeneinrichtung beziehungsweise die Längsachse der Spritzenkappe geneigt ist. Das Verfahren zeichnet sich durch eine hohe Präzision und Funktionssicherheit aus.

Die Erfindung wird im Folgenden anhand der Zeichnung näher erläutert. Es zeigen:
- Figur 1: eine Prinzipskizze einer Röntgeneinrichtung;
- Figur 2: drei Spritzenkappen und deren bei einer Röntgenuntersuchung aufgenommene Abbildung;
- Figur 3: ein erstes Ausführungsbeispiel einer Röntgeneinrichtung;
- Figur 4: ein zweites Ausführungsbeispiel einer Röntgeneinrichtung;
- Figur 5: eine Spritze mit einer schief sitzenden Spritzenkappe in Seitenansicht;
- Figur 6: die Spritze mit der Spritzenkappe gemäß Figur 5 in Draufsicht;
- Figur 7: eine Spritze mit einer korrekt ausgerichteten Spritzenkappe in Seitenansicht und
- Figur 8: die Spritze mit der Spritzenkappe gemäß Figur 7 in Draufsicht.

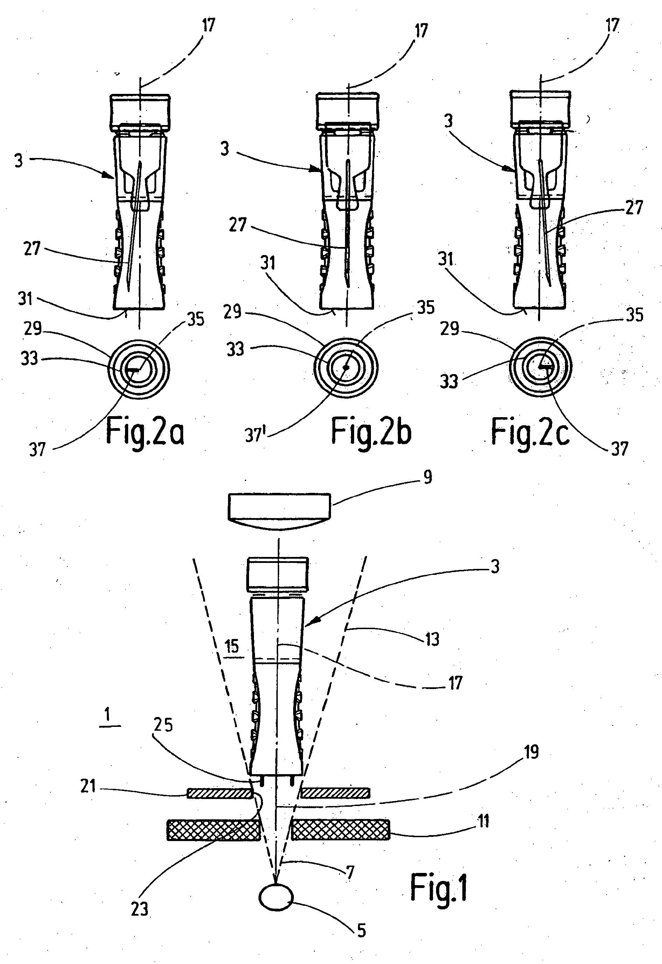

Aus der Prinzipskizze gemäß Figur 1 sind eine Röntgeneinrichtung 1 ersichtlich, die zur Untersuchung einer Spritzenkappe 3 dient, ebenso eine angedeutete Röntgenstrahlungsquelle 5, die Röntgenstrahlen 7 abgibt. In einem Abstand zur Röntgenstrahlungsquelle 5 ist ein Röntgendetektor 9 vorgesehen, der die dort auftreffenden Röntgenstrahlen auswertet. Die Röntgenstrahlen 7 verlaufen in Richtung auf den Röntgendetektor 9 durch einen Kollimator 11, der von der Röntgenstrahlungsquelle 5 ausgehende Strahlung abschirmt und nur einen Teil der Röntgenstrahlen 7 in Richtung auf den Röntgendetektor 9 ungehindert passieren lässt. Es ergibt sich damit ein sich in Richtung auf den Röntgendetektor 9 erweiternder Strahlengang 13. In diesem ist an einem Untersuchungsort 15 die Spritzenkappe 3 angeordnet und zwar derart, dass deren Längsachse 17 mit der Hauptachse 19 des Strahlengangs 13 zusammenfällt. Durch eine derartige Anordnung der Spritzenkappe 3 am Untersuchungsort 15 ist es möglich, diese senkrecht von oben oder unten mit Röntgenstrahlen zu beaufschlagen und somit im Röntgendetektor 9 ein Röntgenbild zu erhalten.

Auf der der Röntgenstrahlungsquelle 5 abgewandten Seite des Kollimators 11 ist ein erstes Referenzelement 21 vorgesehen, das, wie der Kollimator 11, die Breite des Kegels der Röntgenstrahlen 7, die von der Röntgenstrahlungsquelle 5 in Richtung auf den Untersuchungsort 15 verlaufen, einschränkt, um einen definierten kegelförmigen Strahlengang 13 zu realisieren. Das erste Referenzelement 21 weist vorzugsweise eine kreisförmige Öffnung 23 auf, die von dem Röntgendetektor 9 erfasst wird beziehungsweise das dort abgebildete Bild eingrenzt. Das Referenzelement 21 kann ringförmig oder plattenförmig ausgebildet sein, wesentlich ist die kreisförmige Öffnung 23.

In Figur 1 ist zwischen Röntgenstrahlungsquelle 5 und Röntgendetektor 9, hier oberhalb des ersten Referenzelements 21, ein zweites Referenzelement 25 angeordnet, das also, wie der Kollimator 11 und das erste Referenzelement 21 im Bereich der von der Röntgenstrahlungsquelle 5 abgegebenen Röntgenstrahlen 7 angeordnet ist. Von der Röntgenquelle 5 aus gesehen, befindet sich das zweite Referenzelement 25 strahlungsabwärts zum ersten Referenzelement 21. Es ist bei dem hier dargestellten und bevorzugten Ausführungsbeispiel unmittelbar vor dem Untersuchungsort 15 angeordnet. Bei einem anderen, hier nicht dargestellten bevorzugten Ausführungsbeispiel ist das zweite Referenzelement 25 im Strahlengang jenseits des Untersuchungsorts 15 angeordnet, vorzugsweise unmittelbar vor dem Röntgendetektor 9. Das zweite Referenzelement 25 ist ringförmig ausgebildet und dünnwandig, sodass es im Röntgendetektor 9 als dünner Ring abgebildet wird.

Der Außendurchmesser des zweiten Referenzelements 25 ist kleiner als der Innendurchmesser der Öffnung 23. Es ist konzentrisch zur Hauptachse 19 des Strahlengangs 13 angeordnet. Dies gilt auch für die Öffnung 23 des ersten Referenzelements 21.

Figur 2 zeigt drei Spritzenkappen 3, wie sie auch in Figur 1 dargestellt wurden. Hier in Figur 2 ist die Kanüle 27 erkennbar, die sich im Inneren der Spritzenkappe 3 befindet. Bei der linken Darstellung in Figur 2 ist die Kanüle 27 nicht konzentrisch zur Längsachse 17 der Spritzenkappe 3 angeordnet. Sie verläuft vielmehr gegenüber dieser nach links, sodass sie einen sich in Figur 2 nach unten öffnenden Spitzenwinkel mit der Längsachse 17 einschließt. Die Auslenkung der Kanüle 27 kann natürlich auch in anderen Ebenen als der Bildebene von Figur 2 liegen.

Unterhalb der linken Spitzen kappe ist mit a) ein Bild wiedergegeben, wie es von dem in Figur 1 dargestellten Röntgendetektor 9 erfasst wird. Als äußerer Ring 29 wird die von der Oberseite 31 her durchleuchtete Wandung der Spritzenkappe 3 abgebildet. Konzentrisch zum äußeren Ring 29 ist ein dünner Innenring 33 erkennbar, welcher die Abbildung des zweiten Referenzelements 25 darstellt.

Von dem Mittelpunkt 35 des Innenrings 33 nach links verläuft ein schwarzer Strich, der das Abbild 37 der nach links verbogenen Kanüle 27 darstellt.

In der mittleren Darstellung gemäß Figur 2 verläuft die Kanüle 27 konzentrisch zur Längsachse 17 der Spritzenkappe 3. Aus dem darunter liegenden Abbild b) der Spritzenkappe 3 ist ersichtlich, dass in diesem Fall die Kanüle 27 in dem Röntgendetektor 9 als Punkt 37' abgebildet wird, der mit dem Mittelpunkt 35 des Innenrings 33 zusammenfällt, durch den auch die Längsachse 17 der Spritzenkappe 3 verläuft.

Oben rechts in Figur 2 ist die Spritzenkappe 3 mit einer nach rechts ausgelenkten Kanüle 27 ersichtlich. Darunter ist mit c) das Abbild einer derartigen Spritzenkappe 3 im Röntgendetektor 9 wiedergegeben: Es zeigt sich, dass hier ein zwischen dem Mittelpunkt 25 und dem Innenring 33 nach rechts verlaufender Strich zu sehen ist, der das Abbild 37 der Kanüle 27 darstellt.

Nach allem wird deutlich, dass eine korrekt konzentrisch ausgebildete Kanüle 27 als Punkt 37' abgebildet wird, der mit dem Mittelpunkt 35 des Innenrings 33 zusammenfällt. Sobald die Kanüle 27 von der Längsachse 17 der Spritzenkappe 3 ausgelenkt, also verbogen ist, wird sie als Strich abgebildet.

Durch den Innendurchmesser des Innenrings 33 kann ein noch akzeptables Maß einer Auslenkung der Kanüle 27 definiert werden. Ist diese also maximal so weit gegenüber der Längsachse 17 der Spritzenkappe 3 verbogen, dass sie den Innenring 33 berührt, kann die Spritzenkappe 3 als in Ordnung eingestuft werden. Sollte aber das Abbild 37 einer ausgelenkten Kanüle 27 außerhalb des Innenrings 33 enden, würde die zugehörige Spritzenkappe 3 als inakzeptabel ausgesondert.

Die drei Abbildungen a), b) und c) in Figur 2 der in dieser Darstellung wiedergegebenen Spritzenkappe 3 lassen erkennen, dass es auf einfache Weise möglich ist, Spritzenkappen 3 mit konzentrischer Kanüle 27 von solchen zu unterscheiden, bei denen die Kanüle gegenüber der Längsachse 17 der Spritzenkappe 3 verbogen beziehungsweise ausgelenkt ist. Dabei spielt es keine Rolle, ob die Kanüle 27 schräg in einer Spritze montiert ist, auf welche die Spritzenkappe 3 aufgesetzt wird, oder ob die Kanüle 27 beim Aufsetzen der Spritzenkappe 3 aus ihrer ursprünglich korrekten Lage ausgelenkt wird.

Die Röntgeneinrichtung 1 ist also zur Untersuchung von Spritzenkappen 3 einsetzbar, die vormontiert sind und bereits eine Kanüle 27 umfassen. Sie kann aber auch dafür verwendet werden, Spritzen mit einer am Spritzenkörper befestigten Kanüle zu untersuchen, auf die eine Spritzenkappe 3 aufgesetzt ist. Dabei ist es in beiden Fällen möglich, die Spritzenkappe 3 von ihrer Oberseite 31 her zu durchleuchten, oder aber von der gegenüberliegenden Seite. Entscheidend ist allein, dass die Spritzenkappe 3, damit gegebenenfalls auch eine zugehörige Spritze, am Untersuchungsort 15 konzentrisch zur Hauptachse 19 des kegelförmigen Strahlengangs 13 angeordnet ist, sodass die Längsachse 17 der Spritzenkappe 3 mit der Hauptachse 19 zusammenfällt.

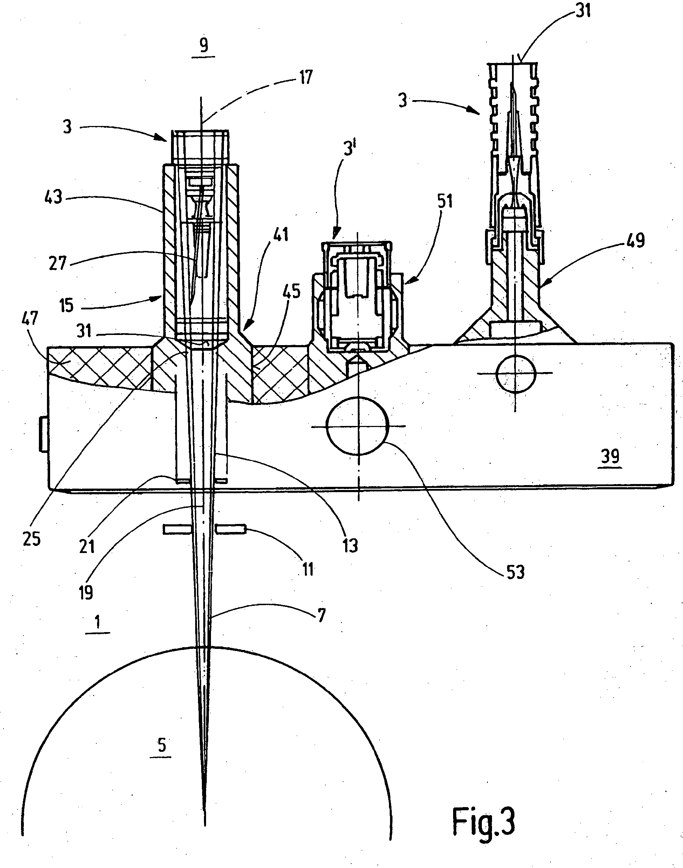

In Figur 3 ist ein erstes Ausführungsbeispiel einer Röntgeneinrichtung 1 wiedergegeben, die nach dem anhand der Figuren 1 und 2 beschriebenen Grundprinzip arbeitet und dazu dient, eine Spritzenkappe 3 mit einer Kanüle 27 zu untersuchen, um festzustellen, ob die Kanüle 27 gegenüber der Längsachse 17 der Spritzenkappe 3 ausgelenkt ist oder nicht.

Aus Figur 3 ist die Röntgenstrahlungsquelle 5 der Röntgeneinrichtung erkennbar, außerdem der Kollimator 11, das erste Referenzelement 21 sowie das zweite Referenzelement 25. Dargestellt sind auch die die Röntgenquelle 5 verlassenden Röntgenstrahlen 7, die durch den Kollimator 11 und das erste Referenzelement 21 treten und einen kegelförmigen Strahlengang 13 ergeben.

Auf der der Röntgenstrahlungsquelle 5 abgewandten Seite des ersten Referenzelements 21 befindet sich bei diesem Ausführungsbeispiel das zweite Referenzelement 25 unmittelbar vor der am Untersuchungsort 15 angeordneten Spritzenkappe 3. Es ist ebenfalls, wie in Figur 1 erläutert, ringförmig ausgebildet.

Oberhalb der Spritzenkappe 3 befindet sich der hier nicht dargestellte Röntgendetektor 9, der ein Abbild der Spritzenkappe 3 liefert.

Bei dem in Figur 3 dargestellten Ausführungsbeispiel der Röntgeneinrichtung 1 ist eine in den Strahlengang 13 eingebrachte Haltevorrichtung 39 vorgesehen, die eine erste Aufnahmeeinheit 41 umfasst. Diese hält die Spritzenkappe 3 am Untersuchungsort 15 in der gewünschten Ausrichtung. Sie weist einen zylindrischen Abschnitt 43 auf, dessen Innendurchmesser so gewählt ist, dass er die Spritzenkappe 3 in seinem Inneren möglichst spielfrei aufnimmt, und deren Mittelachse mit der Hauptachse 19 des Strahlengangs 13 zusammenfällt. Dadurch wird die Spritzenkappe 3 gegenüber dem Strahlengang 13 so ausgerichtet, dass ihre Längsachse 17 mit der Hauptachse 19 des Strahlengangs 13 zusammenfällt. Hier wird die Spritzenkappe 3 von ihrer Oberseite 31 her durchleuchtet. Denkbar ist es aber auch, die erste Aufnahmeeinheit 41 so auszubilden, dass die Spritzenkappe 3, gegebenenfalls mit einer zugehörigen Spritze, umgekehrt am Untersuchungsort 15 gehalten und damit von unten durchleuchtet wird.

Die erste Aufnähmeeinheit 41 ist als Einsatz ausgebildet. Sie kann also in eine entsprechende Ausnehmung 45 im Grundkörper 47 der Haltevorrichtung 39 eingesteckt werden. Auf diese Weise ist es möglich, verschiedene erste Aufnahmeeinheiten 41 mit ein und derselben Haltevorrichtung 39 zu kombinieren.

Aus Figur 3 ist ersichtlich, dass die Haltevorrichtung 39 noch eine zweite Aufnahmeeinheit 49 umfasst, auf welche die Spitzenkappe 3 aufsteckbar ist. Bei dem hier dargestellten Ausführungsbeispiel der Haltevorrichtung 39 ist die zweite Aufnahmeeinheit 49 so ausgebildet, dass bei einer derartigen Halterung die Spritzenkappe 3 mit ihrer Oberseite 31 nach oben von der zweiten Aufnahmeeinheit 49 gehalten wird. Es wird deutlich, dass die Spritzenkappe 3 vor dem Einbringen in den zylindrischen Abschnitt 43 der ersten Aufnahmeeinheit 41 um 180° gedreht werden muss. Dies kann manuell oder durch geeignete Manipulatoren erfolgen.

Auch die zweite Aufnahmeeinheit 49 kann als in die Haltevorrichtung 39 einbringbarer und damit austauschbarer Einsatz ausgebildet sein, um die Halterung 39 an unterschiedliche Spritzenkappen 3 anpassen zu können.

Bei dem in Figur 3 dargestellten Ausführungsbeispiel weist die Haltevorrichtung 39 noch eine dritte Aufnahmeeinheit 51 auf, die anders ausgebildet ist als die anderen Aufnahmeeinheiten. Sie kann dazu dienen, eine andersartige Spritzenkappe 3' oder dergleichen aufzunehmen, die mit Hilfe der Röntgeneinrichtung 1 untersucht werden soll und an den Untersuchungsort 15 verlagert wird, wenn die Röntgenuntersuchung durchgeführt werden soll.

Bevorzugt wird ein Ausführungsbeispiel der Haltevorrichtung 39, die mehrere gleichartige Aufnahmeeinheiten aufweist, die wie die erste Aufnahmeeinheit 41 ausgebildet sind. Beispielsweise könnten derartige Aufnahmeeinheiten kreisförmig im Grundkörper 47 der Haltevorrichtung 39 angeordnet werden. Es ist dann möglich, die Haltevorrichtung 39 nach der Untersuchung einer ersten Spritzenkappe 3 zu drehen, bis die nächste Aufnahmeeinheit mit einer Spritzenkappe 3 am Untersuchungsort 15 angeordnet ist. Sehr wohl können die Aufnahmeeinheiten auch in einer Reihe im Grundkörper 47 der Haltevorrichtung 39 angeordnet sein. In diesem Fall wird dann die Haltevorrichtung 39 einer Transversalbewegung unterworfen, um die Aufnahmeeinheiten mit den Spritzenkappen nacheinander an den Untersuchungsort 15 zu bringen und untersuchen zu können.

Bevorzugt wird die Haltevorrichtung 39 mit einem Datenträger 53 versehen, der hier lediglich angedeutet ist. Bei Untersuchung von Spritzenkappen 3 ist es also möglich, diese chargenweise mittels einer Haltevorrichtung 39 zur Untersuchung einer Röntgeneinrichtung 1 zuzuführen. Die Haltevorrichtung 39 kann dann erfasst und zur Nachverfolgung eines Herstellungsvorgangs gespeichert werden.

Aus Figur 3 ist ersichtlich, dass der Kollimator 11 unterhalb der Haltevorrichtung 39 angeordnet ist und dass das erste Referenzelement 21 sich etwas oberhalb der Unterseite der Haltevorrichtung 39 befindet. Es ist sehr wohl möglich, das erste Referenzelement 21 näher an den Untersuchungsort 15 heranzubringen und beispielsweise an die Stelle des in Figur 3 wiedergegebenen zweiten Referenzelements 25 zu bringen. Dies führt dazu, dass die Strahlung der Röntgenstrahlungsquelle 5 noch besser auf den Untersuchungsort 15 fokussiert wird. In diesem Fall wird dann das zweite Referenzelement 25 - in Strahlungsrichtung gesehen - auf der der Röntgenstrahlungsquelle 5 abgewandten Seite des Untersuchungsorts vor den Röntgendetektor 9 gelegt. Vorzugsweise kann es unmittelbar vor dem Röntgendetektor 9 angeordnet werden, sodass sich ein besonders scharfes Abbild des zweiten Referenzelements 25 ergibt, sodass dieses auch als sehr dünner Ring ausgelegt werden kann.

Schließlich können auch bei allen Röntgeneinrichtungen 1 der hier und im Folgenden besprochenen Art zwei ringförmige Referenzelemente verwendet werden, die konzentrisch zueinander liegen. Es kann damit ein Toleranzbereich definiert werden, innerhalb dessen eine Auslenkung der Kanüle 27 noch akzeptiert werden kann.

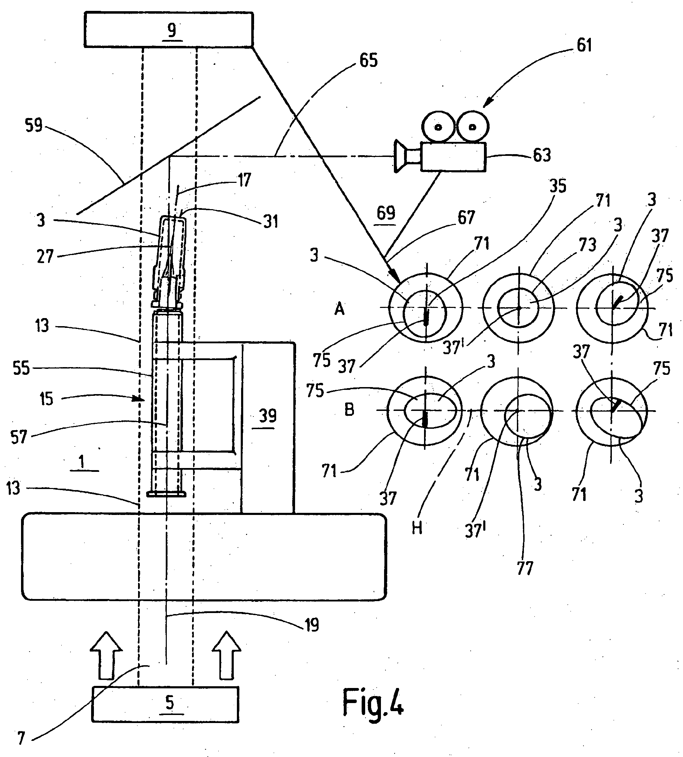

Ein abgewandeltes Ausführungsbeispiel der Röntgeneinrichtung 1 ist in Figur 4 wiedergegeben. Gleiche und funktionsgleiche Teile sind mit gleichen Bezugsziffern versehen. Insofern wird hier auf die vorangegangene Beschreibung verwiesen.

Die Röntgeneinrichtung 1 weist eine Röntgenstrahlungsquelle 5 auf, die Röntgenstrahlen 7 in Richtung auf einen Röntgendetektor 9 abgibt. Ein Strahlengang 13 ist hier lediglich angedeutet. Seine Hauptachse 19 verläuft hier in Figur 4 senkrecht nach oben von der Röntgenstrahlungsquelle 5 durch den Untersuchungsort 15 zum Röntgendetektor 9.

Am Untersuchungsort 15 ist hier eine Spritze 55 zu erkennen, die von einer geeigneten Haltevorrichtung 39 am Untersuchungsort 15 gehalten wird und so ausgerichtet ist, dass ihre Längsachse 57 mit der Hauptachse 19 des Strahlengangs 13 zusammenfällt.

Auf die Spritze 55 ist eine Spritzenkappe 3 aufgesetzt, die hier beispielhaft schräg auf der Spritze 55 sitzt, sodass die Längsachse 17 der Spritzenkappe 3 einen Winkel zur Hauptachse 19 des Strahlengangs 13 aufweist.

Deutlich erkennbar ist, dass bei dem Ausführungsbeispiel gemäß Figur 4 die Spritze 55 und die Spritzenkappe 3 von unten durchleuchtet werden. Die Spritzenkappe 3 ist also, anders als bei den Darstellungen gemäß den Figuren 1 bis 3, so angeordnet, dass ihre Oberseite 31 nach oben weist.

In den Strahlengang 13 ist - von der Röntgenstrahlungsquelle 5 aus gesehen - jenseits des Untersuchungsorts 15 eine einen Spiegel aufweisende Ablenkeinrichtung 59 vorgesehen, die für die Röntgenstrahlen 7 durchlässig ist, die aber Lichtstrahlen ablenkt.

Die Röntgeneinrichtung 1 weist eine Bilderfassungseinheit 61 auf, die beispielsweise eine Kamera 63 umfasst. Durch eine gestrichelte Linie 65 ist angedeutet, dass die Bilderfassungseinheit ein Bild von der Oberseite 31 der Spritzenkappe 3 erfasst.

Die Signale des Röntgendetektors 9 und die der Bilderfassungseinheit 61 werden zusämmengefasst und, wie durch eine Linie 67 angedeutet, gemeinsam ausgewertet, nämlich überlagert. Zur Auswertung der Signale des Röntgendetektors 9 und der Signale der Bilderfassungseinheit 61 wird eine Bildauswertungseinheit 69 verwendet, die hier nicht im Einzelnen dargestellt ist.

Mit A ist die Überlagerung der Bildsignale des Röntgendetektors 9 und der Bilderfassungseinheit 61 angedeutet: Durch einen äußeren Kreis 71 wird eine konzentrisch zur Hauptachse 19 liegende Sollposition vorgegeben, die beispielsweise der Spritze 55 entspricht.

Bei A ist in der Mitte ein konzentrischer innerer Kreis 73 erkennbar. Dieser zeigt das optische Abbild einer Spritzenkappe 3 bei konzentrischer Anordnung, also dann, wenn deren Längsachse 17 mit der Hauptachse 19 des Strahlengangs 13 zusammenfällt. In diesem Fall wird die Kanüle 27 als Punkt 37' abgebildet, wenn diese koaxial zur Längsachse der Spritzenkappe 3 angeordnet ist.

Bei den Abbildungen gemäß A ist links wiederum der äußere Kreis 71 erkennbar. Innen im Kreis 71 ist die Abbildung einer schräg zur Hauptachse 19 des Strahlengangs 13 angeordneten Spritzenkappe 3 erkennbar, die als Ellipse 75 von der Bilderfassungseinheit 61 gesehen wird. Es zeigt sich auch, dass ein vom Mittelpunkt 35 des äußeren Kreises 71 ausgehender Strich erkennbar ist, der das von dem Röntgendetektor 9 erfasste Abbild 37 einer entsprechend ausgelenkten Kanüle 27 darstellt.

Es wird deutlich, dass die Kanüle 37 in der gleichen Richtung ausgelenkt ist, wie die Spritzenkappe 3. Es wird also angenommen, dass die Spritzenkappe 3 und die Kanüle 27 in gleicher Richtung verbogen sind, dass also die Kanüle 27 die Außenwand der Spritzenkappe 3 nirgends durchsticht und dass damit diese Spritzenkappe 3 noch akzeptabel ist.

Entsprechend ist ganz rechts bei den Darstellungen gemäß A ein äußerer Kreis 71 erkennbar. Auch hier wird durch die Bilderfassungseinheit 61 erkannt, dass die Spritzenkappe 3 nach rechts oben ausgelenkt ist. Auch hier ist dies durch eine Ellipse 75 erkennbar. Das Abbild 37 der Kanüle 27 liegt auf der Mittelachse der Ellipse 75. Es wird daher davon ausgegangen, dass die Spritzenkappe 3 und die Kanüle 27 in gleicher Richtung ausgelenkt sind und damit die Kanüle 27, wie bei der linken Darstellung gemäß A, konzentrisch zur Spritzenkappe 3 angeordnet ist. Die mit A gekennzeichneten Darstellungen zeigen also Spritzenkappen 3, die alle in Ordnung sind.

Betrachtet man die Darstellung B in Figur 4, die durch eine Überlagerung der Bilder des Röntgendetektors 3 und der Bilderfassungseinheit 61 entstanden sind, so ist auch hier wieder die äußere Kontur der Spritze 55, die von dem Röntgendetektor 9 erfasst ist, erkennbar und durch einen äußeren Kreis 71 angedeutet. Bei der linken Darstellung gemäß B wird von der Bilderfassungseinheit 61 erkannt, dass die Spritzenkappe 3 nach rechts ausgelenkt ist, sodass die Mittelachse der Ellipse auf einer gedachten Horizontalen H liegt. Der Röntgendetektor 3 zeigt als Strich die Abbildung 37 der Kanüle 27. Diese ist offensichtlich senkrecht nach unten ausgelenkt, also eben nicht in Richtung der Mittelachse der Ellipse 75. Die Bildauswertungseinheit 69 erkennt damit, dass die Spritzenkappe 3 nicht in Ordnung ist.

Eine entsprechende Abweichung der Mittelachse der Ellipse 75 und der damit der Auslenkung der Spritzenkappe 3 von der Auslenkung der Kanüle 27, die durch den Strich 37 vom Röntgendetektor 9 abgebildet wird, zeigt sich auch ganz rechts in der Darstellung B. Auch diese Spritzenkappe 3 wird von der Bildauswertungseinheit 69 als fehlerhaft erkannt.

In der Mitte der Darstellung gemäß B ist Folgendes erkennbar: Durch den äußeren Kreis 61 wird wiederum eine Sollposition vorgegeben oder aber die von dem Röntgendetektor 9 erkannte Wandung der Spritze 55. Ein mit dem Mittelpunkt des Kreises 71 zusammenfallender Punkt 37' zeigt die konzentrisch zur Hauptachse 19 des Strahlengangs 13 ausgerichtete Kanüle 27. Die Bildauswertungseinheit 69 hat erkannt, dass die Spritzenkappe 3 schief sitzt, was durch einen exzentrisch zum Mittelpunkt des Kreises 71 dargestellten Kreis 77 angedeutet ist. Es ist eindeutig, dass die Längsachse 17 der Spritzenkappe 3 nicht mit der der Kanüle 27 zusammenfällt. Die Auswertung des Röntgenbildes mittels des Röntgendetektors 9 und die Erfassung der Spritzenkappe 3 durch die Bilderfassungseinheit 61 ergeben also, dass hier eine Fehlausrichtung der Kanüle 27 innerhalb der Spritzenkappe 3 gegeben ist. Die Bildauswertungseinheit 69 erkennt auch hier ein fehlerhaftes Produkt, sodass dieses ausgesondert werden kann.

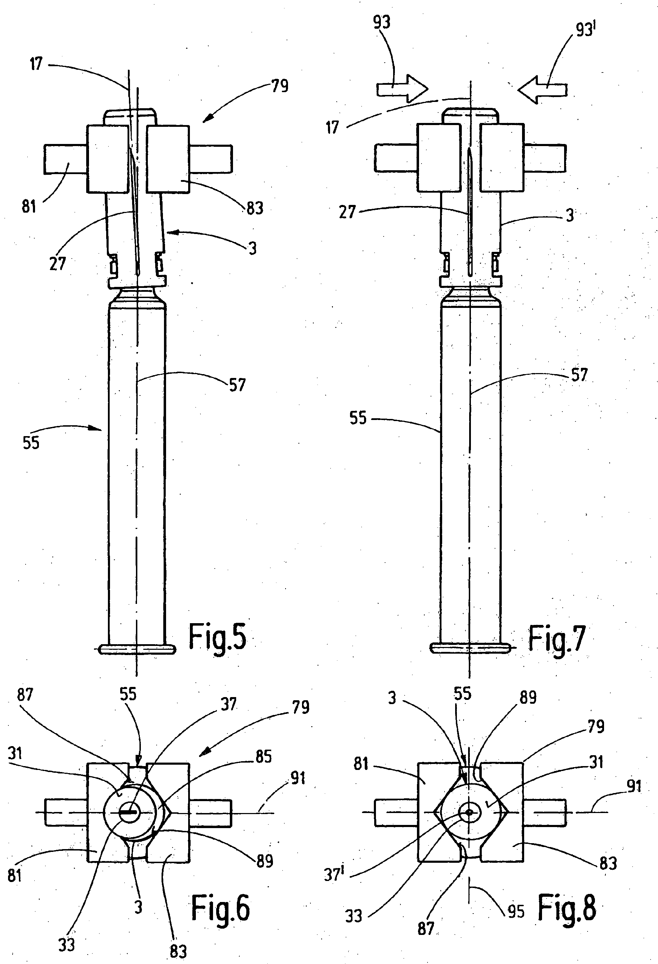

Figur 5 zeigt in Seitenansicht eine Spritze 55 mit einer Spritzenkappe 3. Teile, die anhand der vorangegangenen Figuren bereits erläutert wurden, werden mit gleichen Bezugsziffern versehen.

Die Spritze 55 weist eine Längsachse 57 auf, gegenüber der die Längsachse 17 der Spritzenkappe verschwenkt ist. In Figur 5 ist die Spritzenkappe 3 um einen spitzen Winkel nach links verkippt, weil sie nicht korrekt auf die Spritze 55 aufgesetzt ist. Deshalb ist auch die in der Spritzenkappe liegende, in Figur 5 lediglich angedeutete, Kanüle 27 gegenüber der Mittelachse 57 der Spritze 55 verkippt. Figur 5 zeigt eine Ausrichteinrichtung 79. Diese dient dazu, die Spritzenkappe 3 gegenüber der Spritze 55 korrekt auszurichten, sodass Fluchtungsfehler vermieden werden. Mit Hilfe der Ausrichteinrichtung 79 wird die Spritzenkappe 3 so ausgerichtet, dass deren Längsachse 17 mit der Mittelachse 57 der Spritze 55 fluchtet.

Die Ausrichteinrichtung 79 ist so ausgebildet, dass sie die Spritzenkappe 3 erfasst. Sie weist hier zwei in einem Abstand zueinander angeordnete Greifelemente 81 und 83 auf. Deren Abstand ist so gewählt, dass die Spritze 55 auch mit schief sitzender Spritzenkappe 3 zwischen den Greifelementen 81, 83 angeordnet werden kann.

Figur 6 zeigt die Spritze 55 gemäß Figur 5 in Draufsicht. Erkennbar ist die Spritzenkappe 3. Dabei ist nicht nur deren Oberseite 31 zu sehen, sondern auch Teile ihrer Umfangsfläche 85. Eine optische Bilderfassung vermag also die Schiefstellung der Spritzenkappe 3 ohne Weiteres zu erkennen.

Wird die hier dargestellte Spritze 55 mit der schief sitzenden Spritzenkappe 3 mit einer Röntgeneinrichtung 1 der oben erläuterten Art von oben nach unten oder von unten nach oben durchleuchtet, so wird die Kanüle 27 als Strich dargestellt. Das Abbild 37 der Kanüle 27 ist in Figur 6 eingezeichnet.

Die Röntgeneinrichtung 1 ist so ausgelegt, dass die Schiefstellung der Kanüle 27 an dem strichförmigen Abbild 37 der Kanüle 27 erkannt wird. Denkbar ist es auch, die durch eine Bilderfassungseinheit 61 wiedergegebene Darstellung der Spritzenkappe 3 dazu zu verwenden, deren Schiefstellung zu erkennen. Vorzugsweise werden die Signale des Röntgendetektors 9 und der Bilderfassungseinheit 61 zusammen ausgewertet, insbesondere überlagert.

Das Abbild 37 der Kanüle 27 geht von der Mittelachse 57 der Spritze 55 aus und verläuft von dort im Wesentlichen horizontal nach links. Entsprechend ist rechts von der kreisförmig abgebildeten Oberseite 31 der Spritzenkanüle 3 ein mondsichelförmiger Bereich der Umfangsfläche 85 zu erkennen. Aus den mittels des Röntgendetektors 9 erfassten Abbildung 37 der Kanüle 27 und aus der Lage der Umfangsfläche 85, die in Figur 6 mondsichelförmig wiedergegeben ist, lässt sich feststellen, in welcher Richtung die Spritzenkappe 3 und die Kanüle verschwenkt sind.

Die Ausrichteinrichtung 79 kann nun gezielt eine Gegenbewegung der Spritzenkappe 3 bewirken.

Vorzugsweise weisen die Greifelemente 81 und 83 der Ausrichteinrichtung 79 V-förmige Innenflächen 87 und 89 auf, wobei die Schnittpunkte der Flächenabschnitte der Innenfläche 87 und 89 auf einer gedachten Linie 91 liegen, die durch die Mittelachse 57 der Spritze 55 geht.

Figur 7 zeigt die in Figur 5 wiedergegebene Spritze 55 wiederum in Seitenansicht. Durch Pfeile 93, 93' wird angedeutet, dass die Greifelemente 81 und 83 aufeinander zu bewegt werden, also in Richtung auf die Mittelachse 57 der Spritze 55. Diese wirken so auf die Spritzenkappe 3 ein, dass deren Längsachse 17 mit der Mittelachse 57 fluchtet, sodass sie korrekt auf der Spritze 55 angeordnet ist. Damit liegt auch die Kanüle 27 koaxial zur Längsachse 17 und damit zur Mittelachse 55.

Aus der Draufsicht gemäß Figur 8 ist erkennbar, dass die Innenflächen 87 und 89 in dieser Position die Spritzenkäppe 3 so zentrieren, dass die Kanüle 27 als Punkt 37' von der Röntgeneinrichtung 1 beziehungsweise deren Röntgendetektor 9 abgebildet wird. Eine optische Erfassung der Spritzenkappe 3 würde deren Oberseite 31 als Kreis abbilden. Irgendwelche Bereiche der Umfangsfläche 85 der Spritzenkappe 3 sind hier nicht mehr zu sehen. Das von dem Röntgendetektor 9 gewonnene Abbild der Kanüle und das von einer Bilderfassungseinheit 61 erzeugte Bild der Spritzenkappe 3 zeigen, dass diese nun korrekt ausgerichtet ist.

Durch die aufeinander zu bewegten Greifelemente 81, 83 und durch deren V-förmige Innenflächen 87 und 89 wird die Spritzenkappe 3 exakt gegenüber der Mittelachse 57 der Spritze 55 ausgerichtet. Die Einstellung gegenüber der gedachten Linie 91 erfolgt dadurch, dass die V-förmigen Innenflächen 87 und 89 gerade zu dieser Linie ausgerichtet sind. Werden die Greifelemente 81 und 83 symmetrisch zu einer gedachten Mittelebene 95 bewegt, die durch die Mittelachse 57 der Spritze 55 verläuft und auf der Linie 91 senkrecht steht, so ist auch eine exakte Ausrichtung der Spritzenkappe 3 gegenüber dieser Mittelebene 95 ohne Weiteres gewährleistet. Die Aktivierung der Ausrichteinrichtung kann automatisch erfolgen, wenn der Röntgendetektor 9 und/oder die Bilderfassungseinheit 61 eine Fehlstellung der Spritzenkappe gegenüber der Spritze 55 anzeigen/anzeigt, gewährleistet.

Es sei nochmals darauf hingewiesen, dass die Ausgestaltung der Ausrichteinrichtung 79 auch abgewandelt werden kann. Denkbar ist es beispielsweise die Spritzenkappe 3 durch einen oder mehrere beliebig geformte Greifer unter Auswertung des von Röntgendetektor 9 oder von der Bilderfassungseinheit 61 gewonnenen Bildes so auszurichten, dass die Oberfläche 31 konzentrisch zur Mittelachse 57 der Spritze 55 ausgerichtet ist oder aber die Kanüle 27 als Punkt.37' abgebildet ist.

Sollte es nicht möglich sein, die Oberseite 31 konzentrisch zur Mittelachse 57 auszurichten und gleichzeitig die Kanüle 27 als Punkt auf den Schnittpunkt der Mittelebene 59 mit der Linie 91 abzubilden, ist davon auszugehen, dass die Kanüle 27 nicht konzentrisch zur Spritzenkappe 3 angeordnet ist.

In den Figuren 6 und 8 ist durch eine Linie der Innenring 33 angedeutet, der durch Einsatz eines zweiten Referenzelements 25 im Röntgendetektor 9 abgebildet ist. Dieser dient dazu festzustellen, ob, falls die Kanüle 27 aus der gewünschten Mittellage ausgelenkt ist, diese noch im akzeptablen Bereich liegt oder nicht. Insofern wird auf die Ausführungen zur Bildauswertung gemäß Figur 2 und 4, dort siehe die Abbildungen A und B, verwiesen.

Insgesamt zeigt sich, dass die Durchleuchtung einer Spritzenkappe 3, gegebenenfalls gemeinsam mit einer Spritze 55, mittels einer Röntgeneinrichtung 1 auf einfache Weise die Kontrolle zulässt, ob eine Kanüle 27 korrekt innerhalb einer Spritzenkappe 3 angeordnet ist, zumindest innerhalb eines Toleranzbereichs liegt, der mit Hilfe eines zweiten Referenzelements 25 beziehungsweise des Innenrings 33 definiert und abgelesen werden kann.

In allen Fällen ist eine einzige Röntgenstrahlungsquelle 5 ausreichend, mit der die Spritzenkappe 1 von oben oder von unten durchleuchtet wird, während diese koaxial zum Strahlengang 13 der Röntgenstrahlungsquelle 5 angeordnet ist, während also deren Längsachse 17 mit der Hauptachse 19 des Strahlengangs 13 zusammenfällt.

Aus den Erläuterungen zur Röntgeneinrichtung wird noch Folgendes deutlich: Die Röntgeneinrichtung durchleuchtet die Spritzenkappe 3 entlang ihrer Längsachse 17. Sie kann Glas und Kunststoff von Metall unterscheiden. Auf diese Weise ist es möglich, eine Kanüle 27 aus Metall innerhalb der Spritzenkappe 3 zu erfassen und zwar unabhängig davon, ob die Kanüle als vormontiertes Element der Spritzenkappe 3 vorgesehen oder auf einer Spritze montiert ist, auf welche die Spritzenkappe 3 aufgesetzt ist.

Entscheidendes Kriterium der Röntgeneinrichtung 1 ist also die Möglichkeit, metallische Gegenstände in einer Umgebung zu erfassen, die Glas und/oder Kunststoff enthält.

Die anhand der Figuren erläuterte Röntgeneinrichtung zeichnet sich dadurch aus, dass die zu durchleuchtende Spritzenkappe 3 entlang ihrer Längsachse mit Strahlen beaufschlagt wird. Zusätzlich kann jedoch vorgesehen werden, dass der Untersuchungsort 15 auch seitlich mit Röntgenstrahlung beaufschlagt wird, um zusätzliche Informationen über die Ausrichtung der Kanüle 27 in der Spritzenkappe 3 zu erhalten. Dabei können die Untersuchungsrichtung und die Anzahl der Untersuchungen in einem weiten Rahmen frei gewählt werden, um höchste Sicherheit bezüglich der Produktqualität zu erreichen. In der Regel wird eine am Untersuchungsort angeordnete Spritze beziehungsweise deren Spritzenkappe 3 seitlich, also aus gleicher Höhe durchleuchtet. Es ist aber möglich, die Röntgenquelle so anzuordnen, dass die Spritzenkappe 3 seitlich schräg von unten oder oben untersucht wird. Auch können die Untersuchungen senkrecht zum optimalen Verlauf der Kanüle 27 unter verschiedenen Winkeln durchgeführt werden, um im Zweifelsfall exakte Informationen über den Verlauf der Kanüle 27 innerhalb der Spritzenkappe 3 zu erhalten.

Daher sollen mit dem Begriff "Röntgeneinrichtung" alle Einrichtungen erfasst werden, die die Spritzenkappe 3 mit Hilfe von Strahlen untersuchen, um den Verlauf einer Kanüle 27 innerhalb der Spritzenkappe 3 zu erfassen. Die Erfindung ist also nicht speziell auf Röntgenstrahlung beschränkt, sondern erfasst alle Strahlungen, die eine gleichartige Untersuchung der Spritzenkappe 3 ermöglichen.

## Patentansprüche

1. Röntgeneinrichtung zur Untersuchung von eine Kanüle (27) aufweisenden Spritzenkappen (3), mit
- einer Röntgenstrahlungsquelle (5),
- einem Röntgendetektor (9) und mit einer
- eine Spritzenkappe (3) im Strahlengang (13) an einem Untersuchungsort (15) haltenden Haltevorrichtung (39),
**dadurch gekennzeichnet, dass**
- die Haltevorrichtung (39) mindestens eine erste Aufnahmeeinheit für die Spritzenkappe (3) aufweist, die so ausgebildet ist, dass
- die Spritzenkappe (3) so im Strahlengang (13) angeordnet ist, dass ihre Längsachse (17) mit der Hauptachse (19) des Strahlengangs (13) zusammenfällt.

2. Röntgeneinrichtung nach Anspruch 1, **dadurch gekennzeichnet, dass** zwischen Röntgenstrahlungsquelle (5) und Untersuchungsort (15) mindestens ein Kollimator (11) im Strahlengang (13) angeordnet ist.

3. Röntgeneinrichtung nach Anspruch 1 oder 2, **dadurch gekennzeichnet, dass** zwischen Röntgenstrahlungsquelle (5) und Untersuchungsort (15), vorzugsweise zwischen dem mindestens einen Kollimator (11) und dem Untersuchungsort (15), ein erstes Referenzelement (21) eingebracht ist, das eine vorzugsweise kreisförmige-Öffnung aufweist.

4. Röntgeneinrichtung nach einem der vorhergehenden Ansprüche, **dadurch gekennzeichnet, dass** zwischen Röntgenstrahlungsquelle (5) und Untersuchungsort (15), vorzugsweise zwischen dem mindestens einen Kollimator (11) und dem Untersuchungsort (15) und insbesondere zwischen dem ersten Referenzelement (21) und dem Untersuchungsort (15), ein zweites Referenzelement (25) eingebracht ist.

5. Röntgeneinrichtung nach Anspruch 4, **dadurch gekennzeichnet, dass** das zweite Referenzelement (25) -in Strahlungsrichtung gesehen- unmittelbar vor dem Untersuchungsort (15) angeordnet ist.

6. Röntgeneinrichtung nach Anspruch 4, **dadurch gekennzeichnet, dass** das zweite Referenzelement (25) -in Strahlungsrichtung gesehen- unmittelbar vor dem Röntgendetektor (9) angeordnet ist.

7. Röntgeneinrichtung nach einem der Ansprüche 4 bis 6, **dadurch gekennzeichnet, dass** das zweite Referenzelement (25) als dünner Ring ausgebildet ist.

8. Röntgeneinrichtung nach einem der vorhergehenden Ansprüche, **gekennzeichnet durch** eine Bilderfassungseinheit (61).

9. Röntgeneinrichtung nach einem der vorhergehenden Ansprüche, **gekennzeichnet durch** eine zwischen Untersuchungsort (15) und Röntgendetektor (9) angeordnete Ablenkeinrichtung (59).

10. Röntgeneinrichtung nach Anspruch 8 oder 9, **dadurch gekennzeichnet, dass** das Bild des Röntgendetektors (9) und das Bild der Bilderfassungseinheit (61) einer Bildauswertungseinheit (69) zugeleitet wird.

11. Röntgeneinrichtung nach Anspruch 10, **dadurch gekennzeichnet, dass** die Bilder des Röntgendetektors (9) und der Bilderfassungseinheit (61) überlagert werden.

12. Röntgeneinrichtung nach einem der vorhergehenden Ansprüche, **gekennzeichnet durch** eine im Bereich des Untersuchungsorts (15) angeordnete Ausrichteinrichtung (79).

13. Röntgeneinrichtung nach Anspruch 1, **dadurch gekennzeichnet, dass** die Haltevorrichtung (39) mindestens eine zweite Aufnahmeeinheit (49) aufweist.

14. Röntgeneinrichtung nach Anspruch 13, **dadurch gekennzeichnet, dass** die Haltevorrichtung (39) mindestens eine dritte Aufnahmeeinheit (51) aufweist.

15. Röntgeneinrichtung nach einem der Ansprüche 1, 13 oder 14, **dadurch gekennzeichnet, dass** zumindest die erste Aufnahmeeinheit (41) als in die Haltevorrichtung (39) einbringbarer Einsatz ausgebildet ist.

16. Röntgeneinrichtung nach einem der vorhergehenden Ansprüche, **dadurch gekennzeichnet, dass** mindestens eine zusätzliche Röntgenquelle vorgesehen ist, welche den Untersuchungsort (15) von einer Seite durchleuchtet.

17. Verfahren zur Untersuchung von eine Kanüle aufweisenden Spritzenkappen mittels einer eine Röntgenstrahlungsquelle und einen Röntgendetektor aufweisenden Röntgeneinrichtung, insbesondere einer Röntgeneinrichtung nach einem der Ansprüche 1 bis 16, mit folgenden Schritten:
- eine Spritzenkappe wird im Strahlengang zwischen der Röntgenstrahlungsquelle und dem Röntgendetektor so angeordnet, dass die Längsachse der Spritzenkappe mit der Hauptachse des Strahlengangs zusammenfällt,
- Erfassung eines zumindest eine Abbildung der Kanüle zeigenden Röntgenbilds der Spritzenkappe mittels des Röntgendetektors und
- Vergleich der Form oder Größe der Kanülenabbildung mit einer noch ein akzeptables Maß einer Auslenkung der Kanüle gegenüber der Längsachse der Spritzenkappe definierenden Referenzabbildung.

## Claims

1. An x-ray device for examining syringe caps (3) having a cannula (27), with
- an x-ray source (5),
- an x-ray detector (9) and with a
- a retaining device (39) holding a syringe cap (3) in a beam path (13) at an examination site (15),
**characterized in that**
- the retaining device (39) comprises at least a first receiving unit for the syringe cap (3) being configured such that
- the syringe cap (3) is arranged in the beam path (13) such that its longitudinal axis (17) coincides with the main axis (19) of the beam path (13).

2. The x-ray device according to claim 1, **characterized in that** at least one collimator (11) is arranged in the beam path (13) between the x-ray source (5) and the examination site (15).

3. The x-ray device according to claim 1 or 2, **characterized in that** a first reference element (21) is inserted between the x-ray source (5) and the examination site (15), preferably between the at least one collimator (11) and the examination site (15), the first reference element having a preferably annular opening.

4. The x-ray device according to any one of the preceding claims, **characterized in that** a second reference element (25) is inserted between the x-ray source (5) and the examination site (15), preferably between the at least one collimator (11) and the examination site (15), and in particular between the first reference element (21) and the examination site (15).

5. The x-ray device according to claim 4, **characterized in that** the second reference element (25) is arranged directly in front of the examination site (15) when viewed in the direction of radiation.

6. The x-ray device according to claim 4, **characterized in that** the second reference element (25) is arranged directly in front of the x-ray detector (9) when viewed in the direction of radiation.

7. The x-ray device according to any one of claims 4 to 6, **characterized in that** the second reference element (25) is configured as a thin ring.

8. The x-ray device according to any one of the preceding claims, **characterized by** an image detection unit (61).

9. The x-ray device according to any one of the preceding claims, **characterized by** a deflection device (59) arranged between the examination site (15) and the x-ray detector (9).

10. The x-ray device according to claim 8 or 9, **characterized in that** the image of the x-ray detector (9) and the image of the image detection unit (61) is supplied to an image analysis unit (69).

11. The x-ray device according to claim 10, **characterized in that** the images of the x-ray detector (9) and the image detection unit (61) are overlapped.

12. The x-ray device according to any one of the preceding claims, **characterized by** an alignment device (79) arranged in the region of the examination site (15).

13. The x-ray device according to claim 1, **characterized in that** the retaining device (39) comprises at least a second receiving unit (49).

14. The x-ray device according to claim 13, **characterized in that** the retaining device (39) comprises at least a third receiving unit (51).

15. The x-ray device according to any one of claims 1, 13 or 14, **characterized in that** at least the first receiving device (41) is configured as an insert, which can be inserted into the retaining device (39).

16. The x-ray device according to any one of the preceding claims, **characterized in that** at least one additional x-ray source for transradiating the examination site (15) from one side is provided.

17. Method for examining syringe caps having a cannula by means of an x-ray device comprising an x-ray source and an x-ray detector, in particular by means of an x-ray device according to any one of claims 1 to 16, with the following steps:
- a syringe cap is arranged in the beam path between the x-ray source and the x-ray detector such that the longitudinal axis of the syringe cap coincides with the main axis of the beam path,
- recording at least one x-ray image of the syringe cap by means of the x-ray detector, the image showing at least one representation of the cannula, and
- comparing the shape or size of the representation of the cannula with a reference representation defining a still acceptable deviation of the cannula with respect to the longitudinal axis of the syringe cap.

## Revendications

1. Dispositif à rayons X pour examiner des capuchons de seringue (3) ayant une canule (27), avec
- une source des rayons X (5),
- un détecteur des rayons X (9) et avec un
- dispositif de retenue (39) pour maintenir un capuchon de seringue (3) dans la trajectoire des rayons (13) dans un site d'examen (15),
**caractérisé en ce que**
- le dispositif de retenue (39) présente au moins une première unité de réception pour le capuchon de seringue (3), la première unité étant configurée de telle manière que
- le capuchon de seringue (3) est disposé dans la trajectoire des rayons (13) de telle manière que son axe longitudinal (17) coïncide avec l'axe principal (19) de la trajectoire des rayons (13).

2. Dispositif à rayons X selon la revendication 1, **caractérisé en ce qu'**au moins un collimateur (11) est disposé dans la trajectoire des rayons (13) entre la source des rayons X (5) et le site d'examen (15).

3. Dispositif à rayons X selon la revendication 1 ou 2, **caractérisé en ce qu'**un premier élément de référence (21) est interposé entre la source des rayons X (5) et le site d'examen (15), de préférence entre l'au moins un collimateur (11) et le site d'examen (15), l'élément de référence ayant une ouverture qui est de préférence circulaire.

4. Dispositif à rayons X selon l'une quelconque des revendications précédentes, **caractérisé en ce qu'**un second élément de référence (25) est interposé entre la source des rayons X (5) et le site d'examen (15), de préférence entre l'au moins un collimateur (11) et le site d'examen (15), et notamment entre le premier élément de référence (21) et le site d'examen (15).

5. Dispositif à rayons X selon la revendication 4, **caractérisé en ce que** le second élément de référence (25) est disposé immédiatement avant le site d'examen (15), vu dans la direction des rayons.

6. Dispositif à rayons X selon la revendication 4, **caractérisé en ce que** le second élément de référence (25) est disposé immédiatement avant le détecteur des rayons X (9), vu dans la direction des rayons.

7. Dispositif à rayons X selon l'une quelconque des revendications 4 à 6, **caractérisé en ce que** le second élément de référence (25) est configuré sous forme d'un anneau mince.

8. Dispositif à rayons X selon l'une quelconque des revendications précédentes, **caractérisé par** une unité d'acquisition d'image (61).

9. Dispositif à rayons X selon l'une quelconque des revendications précédentes, **caractérisé par** un dispositif déflecteur (59) disposé entre le site d'examen (15) et le détecteur des rayons X (9).

10. Dispositif à rayons X selon la revendication 8 ou 9, **caractérisé en ce que** l'image du détecteur des rayons (9) et l'image de l'unité d'acquisition d'image (61) sont fournies à une unité d'évaluation d'image (69).

11. Dispositif à rayons X selon la revendication 10, **caractérisé en ce que** les images du détecteur des rayons (9) et de l'unité d'acquisition d'image (61) sont superposées.

12. Dispositif à rayons X selon l'une quelconque des revendications précédentes, **caractérisé par** un dispositif d'alignement (79) disposé dans la région du site d'examen (15).

13. Dispositif à rayons X selon la revendication 1, **caractérisé en ce que** le dispositif de retenue (39) présente au moins une deuxième unité de réception (49).

14. Dispositif à rayons X selon la revendication 13, **caractérisé en ce que** le dispositif de retenue (39) présente au moins une troisième unité de réception (51).

15. Dispositif à rayons X selon l'une quelconque des revendications 1, 13 ou 14, **caractérisé en ce qu'**au moins la première unité de réception (41) est configurée sous forme d'un insert qui peut être inséré dans le dispositif de retenue (39).

16. Dispositif à rayons X selon l'une quelconque des revendications précédentes, **caractérisé en ce qu'**au moins une source des rayons X additionnelle est prévue qui émet des rayons à travers le site d'examen (15) d'un côté.

17. Procédé pour la vérification des capuchons de seringue ayant une canule, au moyen d'un dispositif à rayons X ayant une source des rayons X et un détecteur des rayons X, notamment au moyen d'un dispositif à rayons X selon l'une quelconque des revendications 1 à 16, avec les étapes suivantes :
- agencer un capuchon de seringue dans la trajectoire des rayons entre la source des rayons X et le détecteur des rayons X de telle manière que l'axe longitudinal du capuchon de seringue coïncide avec l'axe principal de la trajectoire des rayons,
- capturer, au moyen du détecteur des rayons X, une image à rayons X du capuchon de seringue montrant au moins une représentation de la canule, et
- comparer la forme ou la taille de la représentation de la canule avec une représentation de référence qui définit une mesure tout juste acceptable pour une déflection de la canule par rapport à l'axe longitudinal du capuchon de seringue.
